(19) **Europäisches Patentamt**
**European Patent Office**
**Office européen des brevets**

(11) **EP 2 378 966 B1**

(12) **EUROPEAN PATENT SPECIFICATION**

(45) Date of publication and mention
of the grant of the patent:
**16.08.2017 Bulletin 2017/33**

(51) Int Cl.:
*A61B 5/08* (2006.01)     *A61B 5/083* (2006.01)
*A61B 5/087* (2006.01)    *A61M 16/00* (2006.01)

(21) Application number: **09801263.6**

(86) International application number:
**PCT/IB2009/055425**

(22) Date of filing: **30.11.2009**

(87) International publication number:
**WO 2010/067254 (17.06.2010 Gazette 2010/24)**

(54) **DETERMINING THE FUNCTIONAL RESIDUAL CAPACITY OF A SUBJECT**

BESTIMMUNG DER FUNKTIONALEN RESTKAPAZITÄT EINER PERSON

DÉTERMINATION DE LA CAPACITÉ FONCTIONNELLE RÉSIDUELLE D UN SUJET

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **09.12.2008 US 120959 P**

(43) Date of publication of application:
**26.10.2011 Bulletin 2011/43**

(73) Proprietor: **Koninklijke Philips N.V.
5656 AE Eindhoven (NL)**

(72) Inventors:
• **ORR, Joseph Allen
Briarcliff Manor, New York 10510-8001 (US)**
• **BREWER, Laura
Briarcliff Manor, New York 10510-8001 (US)**

(74) Representative: **van Velzen, Maaike Mathilde
Philips Intellectual Property & Standards
High Tech Campus 5
5656 AE Eindhoven (NL)**

(56) References cited:
**EP-A2- 1 767 236      WO-A2-2007/026367
DE-A1- 10 046 465**

• **OLEGAARD C ET AL: "ESTIMATION OF
FUNCTIONAL RESIDUAL CAPACITY AT THE
BEDSIDE USING STANDARD MONITORING
EQUIPMENT: A MODIFIED NITROGEN
WASHOUT/WASHIN TECHNIQUE REQUIRING A
SMALL CHANGE OF THE INSPIRED OXYGEN
FRACTION" ANESTHESIA AND ANALGESIA,
WILLIAMS AND WILKINS, BALTIMORE, MD, US,
vol. 101, no. 1, 1 January 2005 (2005-01-01), pages
206-212, XP009063847 ISSN: 0003-2999**

EP 2 378 966 B1

**Description**

BACKGROUND OF THE INVENTION

1. Field of the Invention

The invention relates to the determination of the functional residual capacity of a subject that is being mechanically ventilated.

2. Description of the Related Art

Functional residual capacity is the volume of gas in the lungs at the end of a normal breath. This volume is reduced in some disease states that are seen in mechanically ventilated patients. To address a decrease in functional residual capacity, the Positive End-Expiratory Pressure ("PEEP") of respiratory therapy can be adjusted. In some instances, other parameters of respiratory therapy may also be adjusted based on functional residual capacity.

Conventional ventilation systems that measure functional residual capacity are known. However, these systems generally require a measurement of oxygen consumption. Measurements of oxygen consumption tend to be unreliable at relatively high concentrations of $O_2$ (e.g., oxygen concentrations greater than 80%). Measurements of oxygen consumption also typically require the incorporation of the system that determines functional residual capacity into the overall ventilation system providing respiratory therapy to the subject.

In EP 1 767 236 A2 a ventilator is disclosed for ventilating a patient that assists a clinician in determining a suitable PEEP (positive end expiratory pressure).

WO 2007/026367 A2 discloses a lung volume monitoring method and device for determining functional residual capacity of a patient while ventilating the patient.

SUMMARY OF THE INVENTION

The invention is defined by the claims, all other embodiments are merely exemplary. One aspect of the invention relates to a system configured to determine the functional residual capacity of a subject. In one embodiment the system includes a processor that receives output signals generated by one or more sensors in communication with gas at or near the airway of the subject and executes one or more modules. The one or more modules comprise a concentration module, a composition change module, an alveolar volume module and a functional residual capacity module. The concentration module is configured to determine concentrations of molecular species in gas inhaled and exhaled by the subject from the output signals received by the processor. The composition change module is configured to automatically identify an inhalation composition change from concentrations determined by the concentration module, wherein an inhalation composition change is a change of at least a predetermined magnitude in the concentration of at least one molecular species in gas inhaled by the subject during temporally proximate breaths. The alveolar volume module is configured to determine an alveolar tidal volume of a respiration of the subject from the output signals received by the processor. The functional residual capacity module is configured to determine the functional residual capacity of the subject based on a change in concentrations determined by the concentration module for breaths subsequent to the identified inhalation composition change, a cumulative alveolar ventilation of the subject subsequent to the automatically identified inhalation composition change, and an analysis of the concentration of the molecular species in the gas exhaled by the subject, said analysis comprising implementing a model of the lungs of the subject as a set of n chambers, where n > 1, in a data matching or search algorithm that fits the model of the lungs of the subject to the concentration of the molecular species exhaled by the subject as a function of said cumulative alveolar ventilation of the subject, such that the determination of the functional residual capacity is triggered by the identified inhalation composition change.

Another aspect of the invention relates to a method of determining the functional residual capacity of a subject. In one embodiment, the method comprises determining concentrations of one or more molecular species in gas inhaled by a subject; automatically identifying an inhalation composition change from the determined concentrations, wherein an inhalation composition change is a change of at least a predetermined magnitude in the concentration of at least one molecular species in gas inhaled by the subject during temporally proximate breaths; determining concentrations of one or more molecular species in gas exhaled by the subject in breaths subsequent to the identified inhalation composition change; determining an alveolar ventilation of a respiration of the subject subsequent to the automatically identified inhalation composition change; and determining the functional residual capacity of the subject based on the concentrations of a molecular species in gas exhaled by the subject in breaths subsequent to the identified inhalation composition change, a cumulative alveolar ventilation of the subject subsequent to the automatically identified inhalation composition change, and an analysis of the concentration of the molecular species in the gas exhaled by the subject, said analysis comprising implementing a model of the lungs of the subject as a set of n chambers, where n > 1, in a data matching or search algorithm that fits the model of the lungs of the subject to the concentration of the molecular species exhaled by the subject as a function of said cumulative alveolar ventilation of the subject, such that the determination of the functional residual capacity is triggered by the identified inhalation composition change.

Another aspect of the invention relates to a system configured to determine the functional residual capacity of a subject. In one embodiment, the system comprises means for determining concentrations of one or more molecular species in gas inhaled by a subject; means for automatically identifying an inhalation composition change from the determined concentrations, wherein an inhalation composition change is a change of at least a predetermined magnitude in the concentration of at least one molecular species in gas inhaled by the subject during temporally proximate breaths; means for determining concentrations of one or more molecular species in gas exhaled by the subject in breaths subsequent to the identified inhalation composition change; and means for determining the functional residual capacity of the subject based on the concentrations of a molecular species in gas exhaled by the subject in breaths subsequent to the identified inhalation composition change such that the determination of the functional residual capacity is triggered by the identified inhalation composition change.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.

BRIEF DESCRIPTION OF THE DRAWINGS

FIG. 1 illustrates a system configured to determine the functional residual capacity of a subject, in accordance with one or more embodiments of the invention;

FIG. 2 illustrates a plot of the concentration of $N_2$ in exhaled gas versus cumulative alveolar ventilation, according to one or more embodiments of the invention;

FIG. 3 illustrates a plot showing the implementation of $N_2$ washout to determine functional residual capacity, in accordance with one or more embodiments of the invention;

FIG. 4 illustrates a plot showing the implementation of $N_2$ washout to determine functional residual capacity, according to one or more embodiments of the invention;

FIG. 5 illustrates results obtained using $N_2$ washout to determine functional residual capacity, in accordance with one or more embodiments of the invention; and

FIG. 6 illustrates a method of determining the functional residual capacity of a subject, according to one or more embodiments of the invention.

DETAILED DESCRIPTION OF THE EXEMPLARY EMBODIMENTS

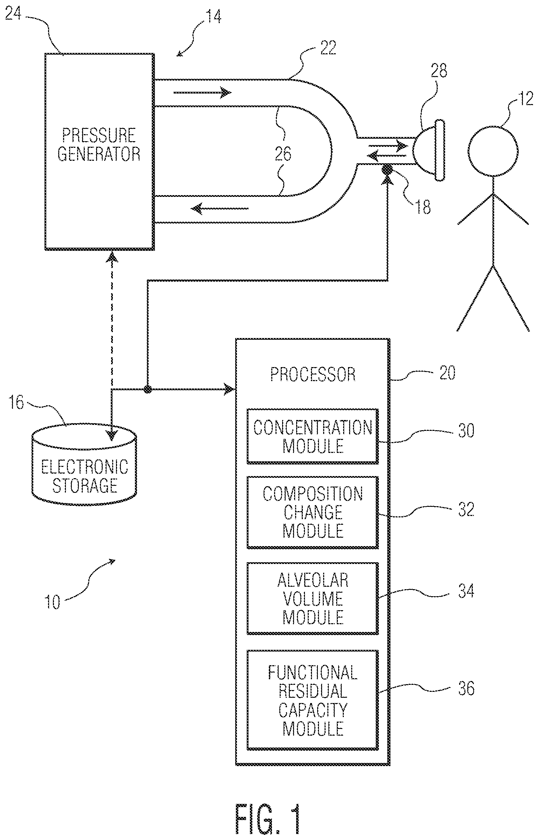

FIG. 1 illustrates a system 10 configured to determine the functional residual capacity of a subject 12. In particular, system 10 determines the functional residual capacity of subject 12 without a measurement of $O_2$ consumption. That is not to say that system 10 is not operable if a measurement of $O_2$ consumption is made, but rather that such a measurement is not required for the determination. The determination of system 10 of functional residual capacity is made in real-time, or near real-time. As used herein, the term "near real-time" refers to processing performed close enough to real time to be of value in providing ongoing therapy to subject 12 from the results of the processing. For example, a determination of functional residual capacity made in near real-time would provide a metric related to the current functional residual capacity of subject 12 that could be used as a feedback parameter for dynamically adjusting one or more aspects of respiratory therapy being provided to subject 12 (e.g., PEEP). In one embodiment, system 10 includes a ventilation system 14, electronic storage 16, sensors 18, and a processor 20.

Ventilation system 14 is configured to mechanically ventilate subject 12. As such, ventilation system 14 includes a gas delivery circuit 22 and a pressure generator 24. In one embodiment, ventilation system 14 is provided integrally with one or more of electronic storage 16, sensors 18, and/or processor 20. In one embodiment, ventilation system 14 is a separate and discrete system from one or more of electronic storage 16, sensors 18, and/or processor 20.

Gas delivery circuit 22 is configured to deliver gas to and receive gas from the airway of subject 12 during ventilation. Gas delivery circuit 22 includes a conduit 26 and an interface appliance 28. Conduit 26 is a flexible conduit that runs between pressure generator 24 and interface appliance 28 to communicate gas therebetween. Interface appliance 28 is configured to deliver gas from conduit 26 to the airway of subject 12, and to receive gas from the airway of subject 12 into conduit 26. Interface appliance 28 may include either an invasive or non-invasive appliance for communicating gas between conduit 26 and the airway of subject 12. For example, interface appliance 28 may include a nasal mask, nasal/oral mask, total face mask, nasal cannula, endotracheal tube, LMA, tracheal tube, and/or other interface appliance. Interface appliance 28 may also include a headgear assembly, such as mounting straps or a harness, for removing and fastening interface appliance 28 to subject 12.

Pressure generator 24 is configured to generate pressure within circuit 22 that pushes gas into and extracts gas from the lungs of subject 12 to mechanically ventilate subject 12. It should be appreciated that although pressure generator 24 is shown in FIG. 1 and referred to in this disclosure as being a single component, pressure generator 24 will typically include two separate sub-systems: one that controllably provides a positive pressure to circuit 22, and one that controllably provides a negative pressure to circuit 22. Each of these separate sub-systems may include a source of pressure (either positive or negative), and one or more valves for controllably placing circuit 14 in communication with the source of pressure. Non-limiting examples of the sources of pressure that may be implemented by one or both of the sub-systems of pressure generator 24 include a wall-gas source, a blower, a pressurized tank or canister of gas, and/or other sources of pressure. In one embodiment, pressure generator 24 also controls the composition of gas provided to subject 12 via circuit 22. For example, in this embodiment, pressure generator may control the concentration of oxygen in the gas provided to subject 12.

In one embodiment, electronic storage 16 comprises electronic storage media that electronically stores information. The electronic storage media of electronic storage 16 may include one or both of system storage that is provided integrally (i.e., substantially non-removable) with system 10 and/or removable storage that is removably connectable to system 10 via, for example, a port (e.g., a USB port, a firewire port, etc.) or a drive (e.g., a disk drive, etc.). Electronic storage 16 may include one or more of optically readable storage media (e.g., optical disks, etc.), magnetically readable storage media (e.g., magnetic tape, magnetic hard drive, floppy drive, etc.), electrical charge-based storage media (e.g., EEPROM, RAM, etc.), solid-state storage media (e.g., flash drive, etc.), and/or other electronically readable storage media. Electronic storage 16 may store software algorithms, information determined by processor 20, information implemented in controlling ventilation system 14, information related to signals generated by sensors 18, and/or other information that enables system 10 to function properly. Electronic storage 16 may be a separate component within system 10, or electronic storage 16 may be provided integrally with one or more other components of system 10 (e.g., processor 20).

In one embodiment, sensors 18 include one or more sensors configured to monitor one or more parameters of the gas within circuit 22. As such, sensors 18 generate output signals that convey information about the one or more parameters of the gas within circuit 22. The one or more parameters may include one or more of a flow rate, a volume, concentrations of one or more molecular species present in the gas, a pressure, a temperature, a humidity, and/or other parameters. In one embodiment, sensors 18 include one or more of a fast $O_2$ sensor, a volumetric capnometry sensor (with outputs related to flow and $CO_2$), a flow rate sensor, a pressure sensor, a capnometer, and/or other sensors. Although sensors 18 are illustrated as being disposed within circuit 22, in one embodiment, at least one of sensors 18 is integrally disposed within pressure generator 24. In this embodiment, output signals generated by sensors 18 may be communicated with a processor external to pressure generator 24 (e.g., processor 20) via a communication port or interface provided on pressure generator 24.

Processor 20 receives output signals generated by sensors 18 (and/or information related to output signals generated by sensors 18). Processor 20 is configured to provide information processing capabilities in system 10. As such, processor 20 may include one or more of a digital processor, an analog processor, a digital circuit designed to process information, an analog circuit designed to process information, a state machine, and/or other mechanisms for electronically processing information. Although processor 20 is shown in FIG. 1 as a single entity, this is for illustrative purposes only. In some implementations, processor 20 may include a plurality of processing units. These processing units may be physically located within the same device, or processor 20 may represent processing functionality of a plurality of devices operating in coordination. For example, in one embodiment, processor 20 represents the processing functionality provided by a processor associated with ventilation system 14 that controls pressure generator 24 and a processor associated with a separate device that determines the functional residual capacity of subject 12.

As is shown in FIG. 1, in one embodiment, processor 20 includes a concentration module 30, a composition change module 32, an alveolar volume module 34, a functional residual capacity module 36, and/or other modules. Modules 30, 32, 34, and/or 36 may be implemented in software; hardware; firmware; some combination of software, hardware, and/or firmware; and/or otherwise implemented. It should be appreciated that although modules 30, 32, 34, and 36 are illustrated in FIG. 1 as being co-located within a single processing unit, in implementations in which processor 20 includes multiple processing units, modules 30, 32, 34, and/or 36 may be located remotely from the other modules. Further, the description of the functionality provided by the different modules 30, 32, 34, and/or 36 described below is for illustrative purposes, and is not intended to be limiting, as any of modules 30, 32, 34, and/or 36 may provide more or less functionality than is described. For example, one or more of modules 30, 32, 34, and/or 36 may be eliminated, and some or all of its functionality may be provided by other ones of modules 30, 32, 34, and/or 36. As another example, processor 20 may include one or more additional modules that may perform some or all of the functionality attributed below to one of modules 30, 32, 34, and/or 36.

Concentration module 30 is configured to determine concentrations of molecular species in gas inhaled and exhaled by subject 12. Concentration module 30 determines this information from the output signals received by processor 20 from sensors 18. The concentrations determined by concentration module 30 include concentrations of one or more of $O_2$, $CO_2$, $N_2$, $H_2O$, and/or other molecular species in gas inhaled by subject 12 in individual inhalations. The concentrations determined by concentration module 30 include concentrations of one or more of $O_2$, $CO_2$, $N_2$, and/or other molecular species in gas exhaled by subject 12 in individual exhalations. In one embodiment, the concentrations determined for $N_2$ are not measured directly by sensors 18. In this embodiment, $N_2$ is assumed to make up all of the gas inhaled or exhaled by subject 12 that is not $O_2$ or $CO_2$. Under this assumption, concentrations of $N_2$ are determined according to the following relationship:

$$ \mathrm{FN}_2 = 1 - \mathrm{FO}_2 - \mathrm{FCO}_2 , \qquad\qquad (1) $$

where $\mathrm{FN}_2$ represents the concentration of $N_2$, $\mathrm{FO}_2$ represents the concentration of $O_2$, and $\mathrm{FCO}_2$ represents the concentration of $CO_2$.

Composition change module 32 is configured to automatically identify an inhalation composition change. An inhalation composition change is a change of at least a predetermined magnitude in the concentration of at least one molecular species in gas inhaled by subject 12 during a predetermined period of time, e.g., defined in units of time, defined as a number of temporally proximate breaths, etc. For example, in one embodiment, composition change module 32 monitors the concentration of $O_2$ inhaled by subject 12, and identifies an inhalation composition change if the concentration of $O_2$ inhaled by subject 12 undergoes a change of a predetermined magnitude or greater. The predetermined magnitude may be, for example, approximately 5%, approximately 7.5%, approximately 10%, approximately 12.5%, approximately 15%, approximately 50%, approximately 70%, and/or other magnitudes.

The concentration of $O_2$ provided to subject 12 by ventilation system 14 for inhalation may be changed during therapy for a variety of reasons. For example, ventilation system 14 may adjust the concentration of $O_2$ provided to subject 12 for inhalation automatically, manually, and/or periodically for monitoring of functional residual capacity, adjustment of partial pressure in mixed venous blood ($PvO_2$), on demand by a caregiver (e.g., during patient suctioning), due to a change in ventilator therapy, for a calibration of an oxygen sensor, and/or for other reasons.

In one embodiment, composition change module 32 identifies inhalation composition changes based on concentrations determined by concentration module 30. In one embodiment, composition change module 32 identifies inhalation composition changes based on measurements of concentrations of one or more molecular species in the gas inhaled by subject 12 from a processor provided in ventilation system 14 to control the composition of gas provided to subject 12 for inhalation.

Alveolar volume module 34 is configured to determine the alveolar tidal volume of the respiration of subject 12. The alveolar tidal volume of respiration is the volume of gas that reaches the pulmonary alveoli in the respiratory system of subject 12 (e.g., the volume of gas that is available for gas exchange with the blood of subject 12 in the lungs). Alveolar volume module 34 makes this determination based on output signals generated by sensors 18 that convey information related to the flow and/or volume of individual inhalations and exhalations by subject 12, and based on concentrations of molecular species in the gases inhaled and exhaled by subject 12. For example, from concentrations of $O_2$ and/or $CO_2$ and volumes of total gas inhaled and exhaled by subject 12 during a given breath, alveolar volume module may determine the alveolar tidal volume of the given breath or cumulative volume from a series of breaths.

Functional residual capacity module 36 is configured to determine the functional residual capacity of subject 12. In one embodiment, functional residual capacity module 36 determines the functional residual capacity of subject 12 from an analysis of the washout or wash-in of one or more molecular species in the gas breathed by subject 12. This analysis is based on concentrations of one or more molecular species present in the gas exhaled by subject 12 determined by concentration module 30 and/or on determinations of alveolar tidal volume made by alveolar volume module 34.

During respiration, $O_2$, $CO_2$, and $N_2$ are inhaled and exhaled from the lungs of subject. If the concentrations of these species in the gas provided to subject 12 for inhalation are held fixed, the concentrations of $O_2$ and $CO_2$ will vary between inhalation and exhalation as these gases are exchanged in the lungs. On the other hand, the concentration of $N_2$, which is not exchanged in the lungs, should be substantially the same in gas that is inhaled and exhaled by subject 12. When the concentrations of these species in gas provided to subject 12 for inhalation are changed during therapy (e.g., an elevation or lowering of $O_2$ for therapeutic purposes), breathing gas having the new composition is met in the lungs with gas that has the previous composition (e.g., the gas held by the functional residual capacity of the lungs). These gases mix, resulting in the exhalation of gas with a concentration of $N_2$ that is different from the inhaled gas. Over the course of the next few breaths, the gas in the functional residual capacity of the lungs having the previous composition is mixed with inhaled gas having the new composition until the level of $N_2$ stabilizes and becomes substantially equal in both inhaled and exhaled gas. This process is referred to as the washout or wash-in of $N_2$. The number of breaths and/or the amount of gas required to stabilize the concentration of $N_2$ following a change in the composition of gas inhaled by subject 12 is a function of the functional residual capacity of subject 12 (e.g., a function of the volume of the gas having the old composition that is held in the lungs of subject 12 at the end of a breath).

As should be appreciated from the foregoing, washout or wash-in of $N_2$ is caused by a change in the composition of gas being inhaled by subject 12. For example, changes in the concentration in $O_2$ in the gas inhaled by subject 12 results in either the washout or wash-in of $N_2$ from the functional residual capacity of the lungs of subject 12. As such, a determination of functional residual capacity by functional residual capacity module 36 is triggered by an identification of an inhalation composition change by composition change module 32.

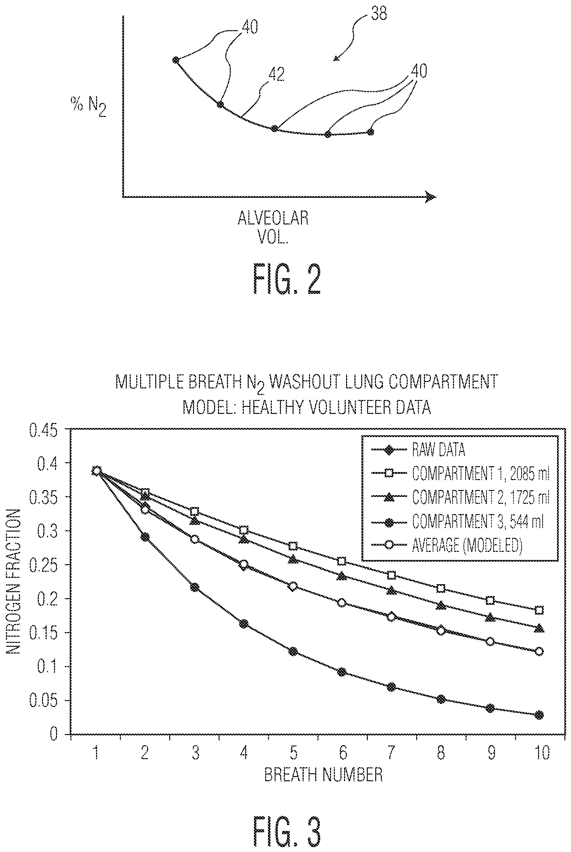

In one embodiment, to determine the functional residual capacity of subject 12 from the washout or wash-in of $N_2$, functional residual capacity module 36 analyzes the concentration of $N_2$ in gas exhaled by subject 12 as a function of cumulative alveolar ventilation for breaths subsequent to an inhalation composition change identified by composition change module 32. By way of illustration, FIG. 2 illustrates a plot 38 of the concentration of $N_2$ in gas exhaled by a subject as a function of cumulative alveolar ventilation of breaths during a washout of $N_2$ from the functional residual capacity of the subject subsequent to an identified inhalation composition change. Plot 38 includes data points 40 that are provided at exhalations subsequent to the identified inhalation composition change, and a line 42 fitted to data points 40.

As can be seen in FIG. 2, the washout of $N_2$ from the functional residual capacity of the subject is generally an exponential decay. In fact, if the lungs of the subject are considered to be a single chamber, plot 38 can be considered as a simple exponential decay, with the volume constant of the decay being the volume of the functional residual capacity of the subject. However, in instances in which the lungs of the subject are not healthy, this model of the lungs (e.g., a single chamber) may not provide an accurate measurement of the functional residual capacity.

In some instances, washout or wash-in of $N_2$ from the lungs of the subject can be modeled as washout or wash-in from a number n chambers, where n is greater than 1 and each chamber has an unknown volume. The functional residual capacity of the n chambers can be determined by a data matching and/or numerical search algorithm that matches plot 38 with data provided by the model including n chambers to determine not only the overall functional residual capacity of the lungs, but also some measure of homogeneity in the volume and ventilation of the chambers (e.g., the residual functional capacities of the individual chambers, etc.). Each of the n chambers is modeled to have a characteristic exponential washout or wash-in. The average of the n washout curves is compared against plot 38.

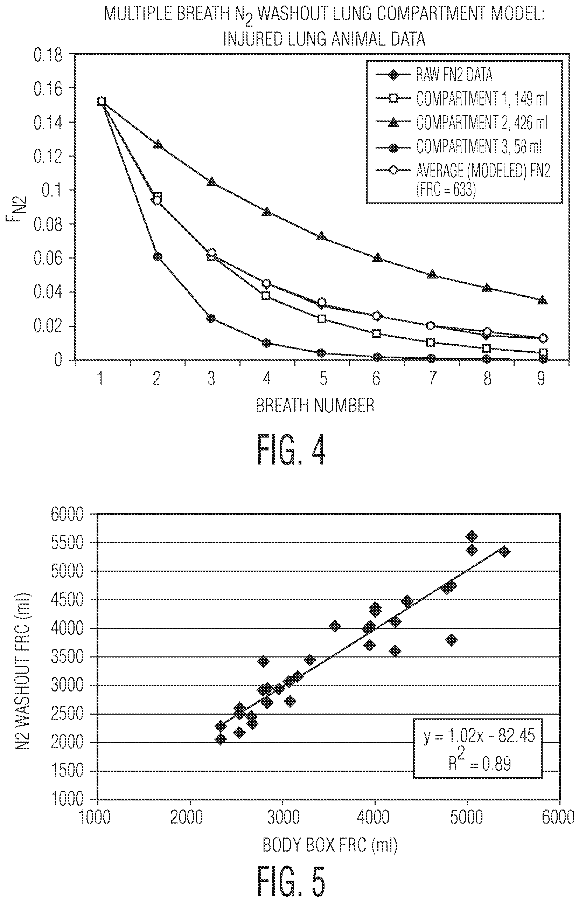

By way of example, FIG. 3 shows a plot that illustrates the approach of modeling the respiratory system as a plurality of separate chambers. In particular, FIG. 3 represents the raw data (the diamond shaped points), which illustrates the exponential decay of the washout of nitrogen from the lungs. FIG. 3 further represents three plots corresponding to separate modeled chambers within the respiratory system of the subject, and then an aggregation of the curves for these separate chambers that matches the raw data. While the subject of the data plotted in FIG. 3 was a healthy subject, with two substantially homogeneous lung chambers and a third smaller chamber representing the rest of the airway, FIG. 4 shows a plot that illustrates the results for an injured (animal) subject. In FIG. 4, the fitting of three separate chamber curves to the raw data provided a result indicating the lung chambers of the injured subject were not roughly equivalent (e.g., an injured lung with a smaller volume than a less injured or non-injured lung).

Returning to FIG. 1, in one embodiment, functional residual capacity module 36 analyzes the concentration of $N_2$ in gas exhaled by subject 12 as a function of the total alveolar ventilation of subject 12 subsequent to an identified inhalation composition change by implementing one or more of the techniques discussed above with respect to plot 38 (shown in FIG. 2 and discussed above). The determination of functional residual capacity by functional residual capacity module 36 may include an overall functional residual capacity of the lungs of subject 12, functional residual capacity in individual chambers of the lungs of subject 12, a metric conveying information about the homogeneity of the functional residual capacity of the lungs of subject 12, and/or other information related to the functional residual capacity of subject 12.

FIG. 5 illustrates experimental results obtained implementing the system described above. In particular, FIG. 5 provides a plot of actual functional residual capacity in a body box, as it is varied between 2550 and 5410 mL, versus measurements of functional residual capacity taken with the above-described system. In making the measurements shown in FIG. 5, changes in inspired oxygen between .5 and 1 were used. As can be seen in FIG. 5, the results correlate well, and the slope of the plot is near unity.

Although FIGS. 3-5 illustrate modeling and results for a wash-out of $N_2$, it should be appreciated that this is not intended to be limiting. The principles illustrated by FIGS. 3-5 and described above are also applicable to other molecular species of gases, and/or for wash-ins as well as wash-outs.

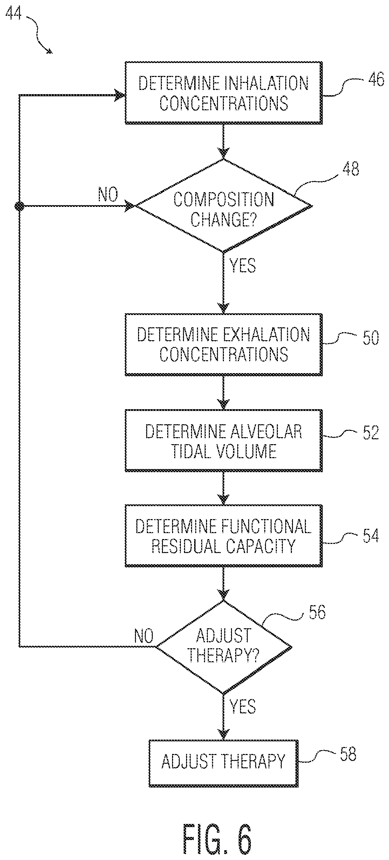

FIG. 6 illustrates a method 44 of determining a functional residual capacity of a subject being mechanically ventilated. The operations of method 44 presented below are intended to be illustrative. In some embodiments, method 44 may be accomplished with one or more additional operations not described, and/or without one or more of the operations discussed. Additionally, the order in which the operations of method 44 are illustrated in FIG. 6 and described below is not intended to be limiting.

In some embodiments, method 44 may be implemented by a system having components similar to those described above with respect to system 10 (shown in FIG. 1). However, this does not limit the disclosure below, as method 44 may be implemented in a variety of other contexts and/or systems than those previously set forth.

At an operation 46, concentrations of one or more molecular species in gas inhaled by the subject are determined. The one or more molecular species may include one or more of $O_2$, $CO_2$, $N_2$, and/or other molecular species. The concentrations of the one or more molecular species may be determined from output signals generated by one or more sensors in communication with the gas and/or from a ventilation system configured to provide gas for inhalation to the subject. In one embodiment, operation 46 is performed by a concentration module that is the same as or similar to concentration module 30 (shown in FIG. 1 and described above).

At an operation 48, a determination is made as to whether an inhalation composition change has occurred, where an inhalation composition change is a change of at least a predetermined magnitude in the concentration of at least one of the molecular species for which the concentration was determined at operation 46. In one embodiment, the magnitude of the composition change is considered in conjunction with a length for which the composition remains different. By way of non-limiting example, in one embodiment a detected change in composition must take place within a predetermined number of breaths or length of time before an inhalation composition change is determined at operation 48. The determination of operation 48 is based on the concentrations determined at operation 46. In one embodiment, operation 48 is performed by a composition change module that is the same as or similar to composition change module 32 (shown in FIG. 1 and described above).

If an inhalation composition change is not identified at operation 48, then method 44 returns to operation 46. If an inhalation composition change is identified at operation 48, then method 44 proceeds to an operation 50.

At operation 50, concentrations of one or more molecular species in gas exhaled by the subject in breaths subsequent to the identified inhalation composition change are determined. The concentrations determined at operation 50 are determined based on output signals of one or more sensors in communication with the gas exhaled by the subject. In one embodiment, operation 50 is performed by the concentration module.

At an operation 52, the cumulative alveolar ventilation of the subject in breaths subsequent to the identified inhalation composition change is determined. The cumulative alveolar ventilation of the subject may be determined from determinations of alveolar tidal volume for individual breaths subsequent to the identified inhalation composition change. The determination of alveolar tidal volume (cumulative and/or individual breath) may be based on concentrations determined at operation 50 and/or 46, and/or based on output signals generated by sensors in communication with gas exhaled by the subject that convey information about the total volume and/or flow of gas into and/or out of the lungs of the subject. In one embodiment, operation 52 is performed by an alveolar volume module that is the same as or similar to alveolar volume module 34 (shown in FIG. 1 and described above).

At an operation 54, a determination of the functional residual capacity of the subject is made. The determination of the functional residual capacity of the subject is made based on an analysis of the washout or wash-in of one or more molecular species from the functional residual capacity of the subject in response to the inhalation composition change identified at operation 48. The analysis of the washout or wash-in of the one or more molecular species includes an analysis of the concentration of the one or more molecular species exhaled by the subject in breaths subsequent to the identified inhalation composition change (e.g., as determined at operation 50) as a function of the cumulative alveolar ventilation of the subject in breaths subsequent to the identified inhalation composition change (e.g., as determined at operation 52). For example, the one or more molecular species for which concentrations are analyzed to determine the functional residual capacity of the subject may include $N_2$. In one embodiment, operation 54 is performed by a functional residual capacity module that is the same as or similar to functional residual capacity module 36 (shown in FIG. 1 and described above).

At an operation 56, a determination is made as to whether the respiratory therapy being provided to the subject should be adjusted based on the functional residual capacity of the subject as determined at operation 54. For example, it may be determined at operation 56 that the PEEP should be adjusted, and/or other aspects of the therapy being provided to the subject may be adjusted based on the functional residual capacity of the subject. To enable the determination of operation 56 to be made, operations 46, 48, 50, 52, and 54 are made in real-time or near real-time. In one embodiment, operation 56 is performed by a processor that controls a ventilation system that is the same as or similar to ventilation system 14 (shown in FIG. 1 and described above). In one embodiment, operation 56 includes providing an alert and/or one or more recommended therapy adjustments to a caregiver and/or clinician, and receiving a command from the caregiver and/or clinician regarding one or more adjustments to be made to the ventilator therapy. In one embodiment, method 44 may be implemented in a monitoring mode. In the monitoring mode, determinations of functional residual capacity are not implemented to control patient ventilation, but may be made to monitor patient health, response to treatment, and/or for other purposes.

If the determination is made at operation 56 that the respiratory therapy should not be adjusted, then method 44 returns to operation 46. If the determination is made at operation 56 that the respiratory therapy being provided to the subject should be adjusted then method 44 proceeds to an operation 58. At operation 58, the respiratory therapy is adjusted in accordance with the determination made at operation 56.

**Claims**

1. A system (10) configured to determine the functional residual capacity of a subject (12), comprising:

  sensors (18) in communication with gas at or near an airway of a subject (12);
  a processor (20) that receives output signals generated by the sensors (18);
  a concentration module (30) configured to determine concentrations of molecular species in gas inhaled and exhaled by the subject, wherein the concentrations are determined from the output signals received by the processor from the sensors;
  a composition change module (32) configured to automatically identify an inhalation composition change from concentrations determined by the concentration module, wherein an inhalation composition change is a change of at least a predetermined magnitude in the concentration of at least one molecular species in gas inhaled by the subject during temporally proximate breaths;
  an alveolar volume module (34) configured to determine an alveolar tidal volume of a respiration of the subject from the output signals received by the processor (20) from the sensors; and
  a functional residual capacity module (36) configured to determine the functional residual capacity of the subject based on

    - a change in concentrations determined by the concentration module for breaths subsequent to the automatically identified inhalation composition change,
    - a cumulative alveolar ventilation of the subject subsequent to the automatically identified inhalation composition change, and
    - an analysis of the concentration of the molecular species in the gas exhaled by the subject as a function of the cumulative alveolar ventilation of the subject (12), said analysis comprising implementing a model of the lungs of the subject as a set of n chambers, where n > 1, in a data matching or search algorithm that fits the model of the lungs of the subject to the concentration of the molecular species exhaled by the subject as a function of said cumulative alveolar ventilation of the subject,

  wherein the determination of the functional residual capacity by the functional residual capacity module (36) is triggered by the automatically identified inhalation composition change.

2. The system (10) of claim 1, wherein the functional residual capacity module (36) is configured to determine the functional residual capacity of the subject (12) based on:

  (a) concentrations of nitrogen in gas exhaled by the subject (12) in breaths subsequent to the identified inhalation composition change; or
  (b) concentrations of oxygen in gas exhaled by the subject (12) in breaths subsequent to the identified inhalation composition change.

3. The system (10) of claim 1, wherein the analysis of the concentration of the molecular species in the gas exhaled by the subject (12) as a function of the cumulative alveolar ventilation of the subject to determine the functional residual capacity of the subject comprises determining the functional residual capacity of the subject based on a volume constant of exponential decay or exponential growth of the concentration of the molecular species as a function of the cumulative alveolar ventilation of the subject in breaths subsequent to the automatically identified inhalation composition change.

4. The system (10) of claim 1, wherein the functional residual capacity module (36) determines the functional residual capacity of the subject (12) in at least near real time.

5. The system (10) of claim 1, wherein the functional residual capacity module (36) determines the functional residual capacity of the subject (12) based on changes in concentrations determined by the concentration module (30) for breaths subsequent to two or more automatically identified inhalation composition changes.

6. A method (44) of determining the functional residual capacity of a subject (12), the method comprising:

   determining concentrations (46) of one or more molecular species in gas inhaled by a subject;
   automatically identifying an inhalation composition change (48) from the determined concentrations, wherein an inhalation composition change is a change of at least a predetermined magnitude in the concentration of at least one molecular species in gas inhaled by the subject during temporally proximate breaths;
   determining concentrations (50) of one or more molecular species in gas exhaled by the subject in breaths subsequent to the automatically identified inhalation composition change;
   determining an alveolar ventilation (52) of a respiration of the subject subsequent to the automatically identified inhalation composition change; and
   determining the functional residual capacity (54) of the subject based on

   - the concentrations of a molecular species in gas exhaled by the subject in breaths subsequent to the automatically identified inhalation composition change,
   - a cumulative alveolar ventilation of the subject subsequent to the automatically identified inhalation composition change, and
   - an analysis of the concentration of the molecular species in the gas exhaled by the subject as a function of the cumulative alveolar ventilation of the subject, said analysis comprising implementing a model of the lungs of the subject as a set ofn chambers, where n > 1, in a data matching or search algorithm that fits the model of the lungs of the subject to the concentration of the molecular species exhaled by the subject as a function of said cumulative alveolar ventilation of the subject,

   wherein the determination of the functional residual capacity is triggered by the automatically identified inhalation composition change.

7. The method (44) of claim 6, wherein further the determination of the functional residual capacity (54) of the subject is based on concentrations of nitrogen in gas exhaled by the subject in breaths subsequent to the automatically identified inhalation composition change.

8. The method (44) of claim 6, wherein the analysis of the concentration of the molecular species in the gas exhaled by the subject (12) as a function of the cumulative alveolar ventilation of the subject to determine the functional residual capacity (54) of the subject comprises determining the functional residual capacity of the subject based on a volume constant of exponential decay of the concentration of the molecular species as a function of the cumulative alveolar ventilation of the subject for breaths subsequent to the automatically identified inhalation composition change.

9. The method (44) of claim 6, further comprising automatically identifying one or more subsequent inhalation composition changes (48) from the determined concentrations; and wherein determining the functional residual capacity (54) of the subject (12) based on the concentrations of a molecular species in gas exhaled by the subject in breaths subsequent to the identified inhalation composition change comprises determining the functional residual capacity of the subject based on the concentrations of the molecular species in gas exhaled by the subject in breaths subsequent to the identified inhalation composition change and in breaths subsequent to the identified subsequent inhalation composition changes.

**Patentansprüche**

1. System (10), das dafür konfiguriert ist, die funktionale Restkapazität einer Person (12) zu bestimmen, und Folgendes umfasst:

   Sensoren (18) in Kommunikationsverbindung mit Gas bei oder nahe einem Atemweg einer Person (12);
   einen Prozessor (20), der durch die Sensoren (18) erzeugte Ausgangssignale empfängt;
   ein Konzentrationsmodul (30), das dafür konfiguriert ist, Konzentrationen von molekularen Spezies in dem durch die Person eingeatmeten und ausgeatmeten Gas zu bestimmen, wobei die Konzentrationen anhand der durch den Prozessor von den Sensoren empfangenen Ausgangssignale bestimmt werden;
   ein Zusammensetzungsänderungsmodul (32), das dafür konfiguriert ist, automatisch eine Einatmungszusammensetzungsänderung anhand der durch das Konzentrationsmodul bestimmten Konzentrationen zu identifizieren, wobei eine Einatmungszusammensetzungsänderung eine Änderung von mindestens einer vorgegebenen Größe in der Konzentration von mindestens einer molekularen Spezies in dem durch die Person während zeitlich naher Atemzüge eingeatmeten Gas ist;

ein alveoläres Volumenmodul (34), das dafür konfiguriert ist, ein alveoläres Tidalvolumen einer Atmung der Person anhand der durch den Prozessor (20) von den Sensoren empfangenen Ausgangssignale zu bestimmen; und

ein funktionales Restkapazitätsmodul (36), das dafür konfiguriert ist, die funktionale Restkapazität der Person zu bestimmen basierend auf:

- einer Änderung in durch das Konzentrationsmodul bestimmten Konzentrationen für Atemzüge im Anschluss an die automatisch identifizierte Einatmungszusammensetzungsänderung,
- einer kumulativen alveolären Ventilation der Person im Anschluss an die automatisch identifizierte Einatmungszusammensetzungsänderung, und
- einer Analyse der Konzentration der molekularen Spezies in dem durch die Person ausgeatmeten Gas als Funktion der kumulativen alveolären Ventilation der Person (12), wobei die genannte Analyse das Implementieren eines Lungenmodells der Person als eine Gruppe von n Kammern, wobei n > 1 ist, in einen Datenabgleich- oder Suchalgorithmus umfasst, der das Lungenmodell der Person an die Konzentration der durch die Person ausgeatmeten molekularen Spezies als eine Funktion der genannten kumulativen alveolären Ventilation der Person anpasst,

wobei das Bestimmen der funktionalen Restkapazität durch das funktionale Restkapazitätsmodul (36) durch die automatisch identifizierte Einatmungszusammensetzungsänderung ausgelöst wird.

2. System (10) nach Anspruch 1, wobei das funktionale Restkapazitätsmodul (36) dafür konfiguriert ist, die funktionale Restkapazität der Person (12) basierend auf Folgendem zu bestimmen:

(a) Konzentrationen von Stickstoff in dem durch die Person (12) ausgeatmeten Gas in Atemzügen im Anschluss an die identifizierte Einatmungszusammensetzungsänderung; oder
(b) Konzentrationen von Sauerstoff in dem durch die Person (12) ausgeatmeten Gas in Atemzügen im Anschluss an die identifizierte Einatmungszusammensetzungsänderung.

3. System (10) nach Anspruch 1, wobei die Analyse der Konzentration der molekularen Spezies in dem durch die Person (12) ausgeatmeten Gas als eine Funktion der kumulativen alveolären Ventilation der Person zum Bestimmen der funktionalen Restkapazität der Person das Bestimmen der funktionalen Restkapazität der Person basierend auf einer Volumenkonstanten des exponentiellen Abklingens oder der exponentiellen Zunahme der Konzentration von molekularen Spezies als eine Funktion der kumulativen alveolären Ventilation der Person in Atemzügen im Anschluss an die automatisch identifizierte Einatmungszusammensetzungsänderung umfasst.

4. System (10) nach Anspruch 1, wobei das funktionale Restkapazitätsmodul (36) die funktionale Restkapazität der Person (12) in mindestens nahezu Echtzeit bestimmt.

5. System (10) nach Anspruch 1, wobei das funktionale Restkapazitätsmodul (36) die funktionale Restkapazität der Person (12) basierend auf Änderungen in den durch das Konzentrationsmodul (30) bestimmten Konzentrationen für Atemzüge im Anschluss an zwei oder mehr automatisch identifizierte Einatmungszusammensetzungsänderungen bestimmt.

6. Verfahren (44) zum Bestimmen der funktionalen Restkapazität einer Person (12), wobei das Verfahren Folgendes umfasst:

Bestimmen von Konzentrationen (46) von einer oder mehreren molekularen Spezies in dem durch eine Person eingeatmeten Gas;
automatisches Identifizieren einer Einatmungszusammensetzungsänderung (48) anhand der bestimmten Konzentrationen, wobei eine Einatmungszusammensetzungsänderung eine Änderung von mindestens einer vorgegebenen Größe in der Konzentration von mindestens einer molekularen Spezies in dem durch die Person während zeitlich naher Atemzüge eingeatmeten Gas ist;
Bestimmen der Konzentrationen (50) von einer oder mehreren molekularen Spezies in dem durch die Person ausgeatmeten Gas in Atemzügen im Anschluss an die automatisch identifizierte Einatmungszusammensetzungsänderung;
Bestimmen einer alveolären Ventilation (52) einer Atmung der Person im Anschluss an die automatisch identifizierte Einatmungszusammensetzungsänderung; und

Bestimmen der funktionalen Restkapazität (54) der Person basierend auf:

- den Konzentrationen einer molekularen Spezies in dem durch die Person ausgeatmeten Gas in Atemzügen im Anschluss an die automatisch identifizierte Einatmungszusammensetzungsänderung,
- einer kumulativen alveolären Ventilation der Person im Anschluss an die automatisch identifizierte Einatmungszusammensetzungsänderung, und
- einer Analyse der Konzentration der molekularen Spezies in dem durch die Person ausgeatmeten Gas als Funktion der kumulativen alveolären Ventilation der Person, wobei die genannte Analyse das Implementieren eines Lungenmodells der Person als eine Gruppe von n Kammern, wobei n > 1 ist, in einen Datenabgleich- oder Suchalgorithmus umfasst, der das Lungenmodell der Person an die Konzentration der durch die Person ausgeatmeten molekularen Spezies als eine Funktion der genannten kumulativen alveolären Ventilation der Person anpasst,

wobei das Bestimmen der funktionalen Restkapazität durch die automatisch identifizierte Einatmungszusammensetzungsänderung ausgelöst wird.

**7.** Verfahren (44) nach Anspruch 6, wobei weiterhin das Bestimmen der funktionalen Restkapazität (54) der Person auf Stickstoffkonzentrationen in dem durch die Person ausgeatmeten Gas in Atemzügen im Anschluss an die automatisch identifizierte Einatmungszusammensetzungsänderung basiert.

**8.** Verfahren (44) nach Anspruch 6, wobei die Analyse der Konzentration der molekularen Spezies in dem durch die Person (12) ausgeatmeten Gas als eine Funktion der kumulativen alveolären Ventilation der Person zum Bestimmen der funktionalen Restkapazität (54) der Person das Bestimmen der funktionalen Restkapazität der Person basierend auf einer Volumenkonstanten des exponentiellen Abklingens der Konzentration von molekularen Spezies als eine Funktion der kumulativen alveolären Ventilation der Person in Atemzügen im Anschluss an die automatisch identifizierte Einatmungszusammensetzungsänderung umfasst.

**9.** Verfahren (44) nach Anspruch 6, weiterhin umfassend das automatische Identifizieren von einer oder mehreren Einatmungszusammensetzungsänderungen (48) anhand der bestimmten Konzentrationen; und wobei das Bestimmen der funktionalen Restkapazität (54) der Person (12) basierend auf den Konzentrationen einer molekularen Spezies in dem durch die Person ausgeatmeten Gas in Atemzügen im Anschluss an die identifizierte Einatmungszusammensetzungsänderung das Bestimmen der funktionalen Restkapazität der Person basierend auf den Konzentrationen der molekularen Spezies in dem durch die Person ausgeatmeten Gas in Atemzügen im Anschluss an die identifizierte Einatmungszusammensetzungsänderung und in Atemzügen im Anschluss an die identifizierten nachfolgenden Einatmungszusammensetzungsänderungen umfasst.

## Revendications

**1.** Système (10) configuré pour déterminer la capacité fonctionnelle résiduelle d'un sujet (12), comprenant :

des capteurs (18) en communication avec un gaz ou à proximité d'une voie respiratoire d'un sujet (12) ;
un processeur (20) qui reçoit des signaux de sortie générés par les capteurs (18) ;
un module de concentration (30) configuré pour déterminer des concentrations d'espèce moléculaire dans un gaz inhalé et exhalé par le sujet, dans lequel les concentrations sont déterminées à partir des signaux de sortie reçus par le processeur depuis les capteurs ;
un module de changement de composition (32) configuré pour identifier automatiquement un changement de composition d'inhalation à partir des concentrations déterminées par le module de concentration, dans lequel un changement de composition d'inhalation est un changement d'au moins une ampleur prédéterminée de la concentration d'au moins une espèce moléculaire dans un gaz inhalé par le sujet pendant des respirations proches dans le temps ;
un module de volume alvéolaire (34) configuré pour déterminer un volume alvéolaire courant d'une respiration du sujet à partir des signaux de sortie reçus par le processeur (20), venant des capteurs ; et
un module de capacité fonctionnelle résiduelle (36) configuré pour déterminer la capacité fonctionnelle résiduelle du sujet sur la base

- d'un changement des concentrations déterminé par le module de concentration des respirations après le changement identifié de façon automatique de la composition d'inhalation,
- d'une ventilation alvéolaire cumulative du sujet après le changement identifié de façon automatique de la composition d'inhalation, et
- d'une analyse de la concentration de l'espèce moléculaire dans le gaz exhalé par le sujet en fonction de la ventilation alvéolaire cumulative du sujet (12), ladite analyse comprenant la mise en oeuvre d'un modèle des poumons du sujet en tant qu'ensemble de n chambres, où n > 1, dans un algorithme d'appariement de données ou de recherche qui correspond au modèle des poumons du sujet par rapport à la concentration de l'espèce moléculaire exhalée par le sujet en fonction de ladite ventilation alvéolaire cumulative du sujet,

dans lequel la détermination de la capacité fonctionnelle résiduelle par le module de capacité fonctionnelle résiduelle (36) est déclenchée par le changement identifié de façon automatique de la composition d'inhalation.

2. Système (10) selon la revendication 1, dans lequel le module de capacité fonctionnelle résiduelle (36) est configuré pour déterminer la capacité fonctionnelle résiduelle du sujet (12) sur la base :

(a) des concentrations d'azote dans le gaz exhalé par le sujet (12) dans les respirations après le changement identifié de la composition d'inhalation ; ou
(b) des concentrations d'oxygène dans le gaz exhalé par le sujet (12) dans les respirations après le changement identifié de la composition d'inhalation.

3. Système (10) selon la revendication 1, dans lequel l'analyse de la concentration de l'espèce moléculaire dans le gaz exhalé par le sujet (12) en fonction de la ventilation alvéolaire cumulative du sujet pour déterminer la capacité fonctionnelle résiduelle du sujet comprend la détermination de la capacité fonctionnelle résiduelle du sujet sur la base d'une constante volumique de diminution exponentielle ou de croissance exponentielle de la concentration de l'espèce moléculaire en fonction de la ventilation alvéolaire cumulative du sujet dans des respirations après le changement identifié de façon automatique de la composition d'inhalation.

4. Système (10) selon la revendication 1, dans lequel le module de capacité fonctionnelle résiduelle (36) détermine la capacité fonctionnelle résiduelle du sujet (12) pratiquement en temps réel.

5. Système (10) selon la revendication 1, dans lequel le module de capacité fonctionnelle résiduelle (36) détermine la capacité fonctionnelle résiduelle du sujet (12) sur la base de changements de concentrations déterminés par le module de concentration (30) pour les respirations après les deux changements ou plus, identifiés de façon automatique, de la composition d'inhalation.

6. Procédé (44) de détermination de la capacité fonctionnelle résiduelle d'un sujet (12), le procédé comprenant :

la détermination de concentrations (46) d'une ou plusieurs espèces moléculaires dans un gaz inhalé par un sujet ;
l'identification automatique d'un changement de composition d'inhalation (48) à partir des concentrations déterminées, dans lequel un changement de composition d'inhalation est un changement d'au moins une ampleur prédéterminée de la concentration d'au moins une espèce moléculaire dans un gaz inhalé par le sujet pendant des respirations proches dans le temps ;
la détermination de concentrations (50) d'une ou plusieurs espèces moléculaires dans un gaz exhalé par le sujet dans des respirations après le changement identifié de façon automatique de la composition d'inhalation ;
la détermination d'une ventilation alvéolaire (52) d'une respiration du sujet après le changement identifié de façon automatique de la composition d'inhalation ; et
la détermination de la capacité fonctionnelle résiduelle (54) du sujet sur la base

- des concentrations d'une espèce moléculaire dans un gaz exhalé par le sujet dans des respirations après le changement identifié de façon automatique de la composition d'inhalation,
- d'une ventilation alvéolaire cumulative du sujet après le changement identifié de façon automatique de la composition d'inhalation, et
- d'une analyse de la concentration de l'espèce moléculaire dans le gaz exhalé par le sujet en fonction de la ventilation alvéolaire cumulative du sujet, ladite analyse comprenant la mise en oeuvre d'un modèle des poumons du sujet en tant qu'ensemble de n chambres, où n > 1, dans un algorithme d'appariement de données ou de recherche qui correspond au modèle des poumons du sujet par rapport à la concentration de l'espèce moléculaire exhalée par le sujet en fonction de ladite ventilation alvéolaire cumulative du sujet,

dans lequel la détermination de la capacité fonctionnelle résiduelle est déclenchée par le changement identifié de façon automatique de la composition d'inhalation.

7. Procédé (44) selon la revendication 6, dans lequel en outre la détermination de la capacité fonctionnelle résiduelle (54) du sujet est basée sur des concentrations d'azote dans le gaz exhalé par le sujet dans des respirations après le changement identifié de façon automatique de la composition d'inhalation.

8. Procédé (44) selon la revendication 6, dans lequel l'analyse de la concentration de l'espèce moléculaire dans le gaz exhalé par le sujet (12) en fonction de la ventilation alvéolaire cumulative du sujet pour déterminer la capacité fonctionnelle résiduelle (54) du sujet comprend la détermination de la capacité fonctionnelle résiduelle du sujet sur la base d'une constante volumique de diminution exponentielle de la concentration de l'espèce moléculaire en fonction de la ventilation alvéolaire cumulative du sujet pour des respirations après le changement identifié de façon automatique de la composition d'inhalation.

9. Procédé (44) selon la revendication 6, comprenant en outre l'identification automatique d'un ou plusieurs changements (48) consécutifs de la composition d'inhalation à partir des concentrations déterminées ; et dans lequel la détermination de la capacité fonctionnelle résiduelle (54) du sujet (12) sur la base des concentrations d'une espèce moléculaire dans un gaz exhalé par le sujet dans des respirations consécutives au changement identifié de la composition d'inhalation comprend la détermination de la capacité fonctionnelle résiduelle du sujet sur la base des concentrations de l'espèce moléculaire dans un gaz exhalé par le sujet dans des respirations consécutives au changement identifié de la composition d'inhalation et dans des respirations consécutives aux changements identifiés consécutifs de la composition d'inhalation.

FIG. 1

FIG. 2

MULTIPLE BREATH $N_2$ WASHOUT LUNG COMPARTMENT
MODEL: HEALTHY VOLUNTEER DATA

FIG. 3

MULTIPLE BREATH $N_2$ WASHOUT LUNG COMPARTMENT MODEL:
INJURED LUNG ANIMAL DATA

Legend:
- RAW FN2 DATA
- COMPARTMENT 1, 149 ml
- COMPARTMENT 2, 426 ml
- COMPARTMENT 3, 58 ml
- AVERAGE (MODELED) FN2 (FRC = 633)

X-axis: BREATH NUMBER

Y-axis: $F_{N2}$

FIG. 4

X-axis: BODY BOX FRC (ml)

Y-axis: N2 WASHOUT FRC (ml)

$y = 1.02x - 82.45$
$R^2 = 0.89$

FIG. 5

FIG. 6

**EP 2 378 966 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1767236 A2

- WO 2007026367 A2